# EUROPÄISCHE PATENTSCHRIFT

(11) **EP 2 570 350 B1**
(45) Veröffentlichungstag und Bekanntmachung des Hinweises auf die Patenterteilung: **24.02.2016**
(21) Anmeldenummer: 12005714.6
(22) Anmeldetag: 07.08.2012
(51) Int. Cl.: B65B 3/00, B65B 43/42, B65B 61/28

(54) **Verfahren und Vorrichtung zum Füllen und Verschließen von pharmazeutischen Objekten**
Method and device for filling and sealing pharmaceutical objects
Procédé et dispositif de remplissage et de fermeture d'objets pharmaceutiques

(30) Priorität: 15.09.2011 DE 102011113358
(43) Veröffentlichungstag der Anmeldung: 20.03.2013
(73) Patentinhaber: GRONINGER & CO. GMBH, D-74564 Crailsheim (DE)
(72) Erfinder:
(74) Vertreter: Witte, Weller & Partner Patentanwälte mbB

(56) Entgegenhaltungen:
- WO-A1-2009/153018
- DE-A1- 10 028 823
- DE-A1-102005 006 733
- DE-A1-102009 027 452

## Beschreibung

Die Erfindung bezieht sich auf ein Verfahren und auf eine Vorrichtung zum Füllen und Verschließen von pharmazeutischen Objekten, z. B. Spritzen, Vials, Zylinderampullen od. dgl., die in parallelen Reihen in Nestern angeordnet sind.

Es ist ein Verfahren vorgenannter Art bekannt, bei dem die Verarbeitung/Bearbeitung von Objekten überwiegend in dem Zustand erfolgt, bei dem die Objekte sich in den Nestern befinden. Als Nester werden üblicherweise etwa plattenförmige Aufnahmeeinrichtungen bezeichnet, die Aufnahmen enthalten, in denen die Objekte z. B. formschlüssig aufgenommen werden. Diese Verfahrensweise, bei der die Objekte in den Nestern bei der Bearbeitung verbleiben, ist an sich von Vorteil, weil die Objekte in den Nestern sortiert und orientiert vorliegen und in dieser Weise direkt verarbeitet werden können. Bei den Bearbeitungsstationen, bei denen die Objekte einzeln verarbeitet werden müssen, z. B. bei Wiegestationen, müssen jedoch die Objekte jeweils aus dem Nest gehoben werden, erforderlichenfalls auch mehrfach, und wieder in das Nest zurückgesetzt werden. Es gibt eine Verfahrensweise, bei der die Objekte zunächst aus dem Nest gehoben werden, sodann Bearbeitungsstationen wie Wiegestation, Füllstation und Wiegestation durchlaufen und danach wieder in das Nest zurückgegeben werden. Bei einer anderen Methode werden die Objekte aus dem Nest gehoben, in der Wiegestation gewogen, sodann in das Nest zurückgegeben, dann in der Füllstation gefüllt, sodann wieder aus dem Nest gehoben und gewogen und danach wieder zurück in das Nest gebracht. In der Verschließstation, wo Verschlussstopfen eingesetzt werden, befinden sich die Objekte im Nest. Das mehrfache Ausheben der Objekte aus den Nestern und erneute Einsetzen in diese ist nachteilig. Zum einen führt dies zu entsprechend langen Bearbeitungszeiten. Zum anderen können sich die Objekte gegenseitig berühren. Die mehrfachen Handhabungsbewegungen der Objekte können zu Produktschädigungen führen.

Druckschrift DE 100 28 823 A1 zeigt ein Verfahren zum Befüllen, Gefriertrocknen und Verschließen von Fertigspritzen, bei welchem die zur Abfüllung vorbereiteten, in Magazinen angeordneten Spritzenzylinder auf einem Magazinband bereitgestellt und in einer Übergabestation auf ein in sich geschlossenes Transportband überstellt werden. Bei Doppelkammerspritzen wird dann der Mittelstopfen gesetzt und anschließend der Spritzenzylinder hinsichtlich seiner Längsachse gewendet. Anschließend erfolgt die Befüllung der kanülenseitigen Kammer und sodann die Vormontage des Verschlussteils, worauf die Spritzenzylinder zur Überstellung in den Gefriertrockner zurück in das Magazin verbracht werden. Nach Abschluss der Gefriertrocknung und Schließen der Spritzenzylinder werden diese wieder an das Transportband übergeben, worauf bei entgegengesetzter Laufrichtung des Transportbandes nach erneutem Wenden der Spritzenzylinder die Befüllung mit dem für die Rekonstitution der gefriergetrockneten Substanz benötigten Lösungsmittel erfolgt. Sodann wird der Endstopfen gesetzt und schließlich werden die Spritzenzylinder zur weiteren Bearbeitung wieder in das Magazin überstellt.

Druckschrift DE 10 2009 027 452 A1 zeigt eine Vorrichtung zum Füllen und Verschließen von pharmazeutischen Behältnissen, wobei die Behältnisse in einem insbesondere wannenförmigen Aufnahmebehälter aufgenommen sind, in dem ein aus dem Aufnahmebehälter entnehmbares Trägerelement eingesetzt ist, in dem die Behältnisse in mehreren Reihen neben- und hintereinander in Aufnahmen des Trägerelements angeordnet sind, mit einer ersten Handhabungseinheit zum Entnehmen des Trägerelements aus dem Aufnahmebehälter, einer Füll- und Verschließeinrichtung für die Behältnisse und einer zweiten Handhabungseinheit zum Wiedereinsetzen des Trägerelements in den auf einer Fördereinrichtung mit dem Trägerelement mitgeförderten Aufnahmebehälter. Erfindungsgemäß ist es vorgesehen, dass die Behältnisse als Spritzkörper oder als mit Bördelkappen zu versehende Behältnisse wie Vials oder Zylinderampullen ausgebildet sind und dass der zweiten Handhabungseinrichtung eine Bördeleinrichtung zugeordnet ist, die beim Handhaben von mit Bördelkappen zu versehenen Behältnissen von der zweiten Handhabungseinrichtung angesteuert wird, so dass die zweite Handhabungseinrichtung die Behältnisse zunächst der Bördeleinrichtung zuführt und anschließend die zuvor mittels der Bördeleinrichtung verschlossenen Behältnisse wieder in das Trägerelement einsetzt.

Die WO 2009/153018A1 beschreibt ein Verfahren zum Befüllen von Doppelkammersystemen in vorsterilisierbaren Trägersystemen, das die Schritte umfasst: Bereitstellen von mindestens einem gewaschenen, silikonisierten und sterilisierten Doppelkammersystem mit je einem die beiden Kammern voneinander trennenden Trennelement in einem Magazin, welches das mindestens eine Doppelkammersystem aufnimmt, wobei das Magazin in einem mit einem Verschlusselement versiegelten Behälter angeordnet ist; Einschleusen des Behälters in einen Reinraum; Öffnen des Behälters und Befüllen einer ersten Kammer des mindestens einen Doppelkammersystems; Verschließen der ersten Kammer mit einem gasdurchlässigen Verschlusselement; Gefriertrocknen der in der ersten Kammer enthaltenen Lösung; Verschließen der ersten Kammer mit einem Verschlusselement; Befüllen einer zweiten Kammer des mindestens einen Doppelkammersystems; Verschließen der zweiten Kammer; Ausschleusen aus dem Reinraum.

Die DE 10 2005 006 733 A1 betrifft eine Behälter-Befüllvorrichtung für die Befüllung von Behältern mit fließfähigen Produkten, die eine Füllstation und eine Transportstrecke aufweist, die die Behälter in einem genau bestimmten Abstand voneinander der Füllstation zuführt. Die Transportstrecke enthält zwei gegenläufige Förderschnecken. In die Transportstrecke ist vor und hinter der Füllstation je eine Wägevorrichtung eingeschaltet. In den entsprechenden Wägebereichen sind die Förderschnecken so reduziert, dass sie die Behälter freigeben. Diese werden dann auf einem Einlaufband beruhigt und auf ein Wägeband gebracht, wo ihr Gewicht festgestellt wird.

Der Erfindung liegt die Aufgabe zu Grunde, ein Verfahren und eine Vorrichtung zum Füllen und Verschließen von pharmazeutischen Objekten zu schaffen, die eine Leistungssteigerung beim Füllen und Verschließen ermöglichen bei sehr großer Genauigkeit und 100 % Kontrolle der Füllmenge und die durch Reduzierung von Handhabungsbewegungen etwaige Produktschädigungen ausschließen.

Die Aufgabe wird durch ein Verfahren gemäß Anspruch 1 und eine Vorrichtung gemäß Anspruch 10 gelöst.

Die Objekte werden an einem Eingang einer Fördereinrichtung in einer Entnahmestation den Nestern entnommen und in die Fördereinrichtung eingebracht, mittels der Fördereinrichtung werden die Objekte entlang eines geraden Abschnitts der Transportstrecke nacheinander einer ersten Wiegestation, in der die Objekte gewogen werden, einer Füllstation, in der die Objekte befüllt werden, und einer zweiten Wiegestation zugeführt, in der die befüllten Objekte wiederum gewogen werden. Danach werden die befüllten Objekte einer Verschließstation und/oder Bördelstation zugeführt, in der die befüllten Objekte verschlossen werden. Die befüllten Objekte können dann aus der Transportstrecke abgegeben werden. Die zu bearbeitenden Objekte sind vorsterilisiert. Die Bearbeitung erfolgt aseptisch.

Gemäß dem Verfahren werden die Objekte am Anfang der Fördereinrichtung aus den Nestern entnommen, dann werden die Objekte komplett mit allen Bearbeitungsschritten verarbeitet und am Ende werden die Objekte entweder wieder in die Nester eingesetzt oder am Ende vereinzelt aus der Fördereinrichtung entfernt.

Die erfindungsgemäße Methode hat umso mehr Vorteile, je mehr Bearbeitungsstationen die Vorrichtung aufweist. In vorteilhafter Weise erfolgt ein berührungsfreier Transport der Objekte beim Transport mittels der Fördereinrichtung entlang den Bearbeitungsstationen. Es ist eine 100 %ige Kontrolle der Füllmenge beim Füllen in der Füllstation möglich, ohne größere Leistungseinbußen. Bei Objekten in Form von Vials kann zusätzlich zum

Verschließen in der Verschließstation durch Einbringen eines Stopfens auch noch ein Bördeln von Bördelkappen erfolgen. Da die Objekte nur einmal beim Entnehmen aus den Nestern und einmal beim erneuten Einsetzen in die Nester nach Durchlauf aller Bearbeitungsschritte gehandhabt werden müssen, finden weniger Handhabungsbewegungen statt, so dass die Gefahr etwaiger Produktschädigungen entfällt oder zumindest weitestgehend gebannt ist. Von Vorteil bei der beschriebenen Einzelverarbeitung der Objekte ist ferner, dass freie Sicht auf das jeweilige Objekt besteht. Dies erlaubt optische Prüfverfahren, z. B. die Messung der Einsetztiefe beim Verschließen durch Stopfensetzen.

Weitere vorteilhafte Verfahrensmerkmale und Ausgestaltungen des Verfahrens gemäß Anspruch 1 ergeben sich aus den Unteransprüchen.

Weitere Einzelheiten und Vorteile der Erfindung sind der Beschreibung zu entnehmen.

Die Erfindung ist nachfolgend anhand von in den Zeichnungen gezeigten Ausführungsbeispielen näher erläutert. Es zeigen:
- Fig. 1: eine schematische Darstellung einer Vorrichtung zum Füllen und Verschließen von pharmazeutischen Objekten, gemäß einem ersten Ausführungsbeispiel,
- Fig. 2: eine schematische Darstellung etwa entsprechend Fig. 1 einer Vorrichtung gemäß einem zweiten Ausführungsbeispiel.

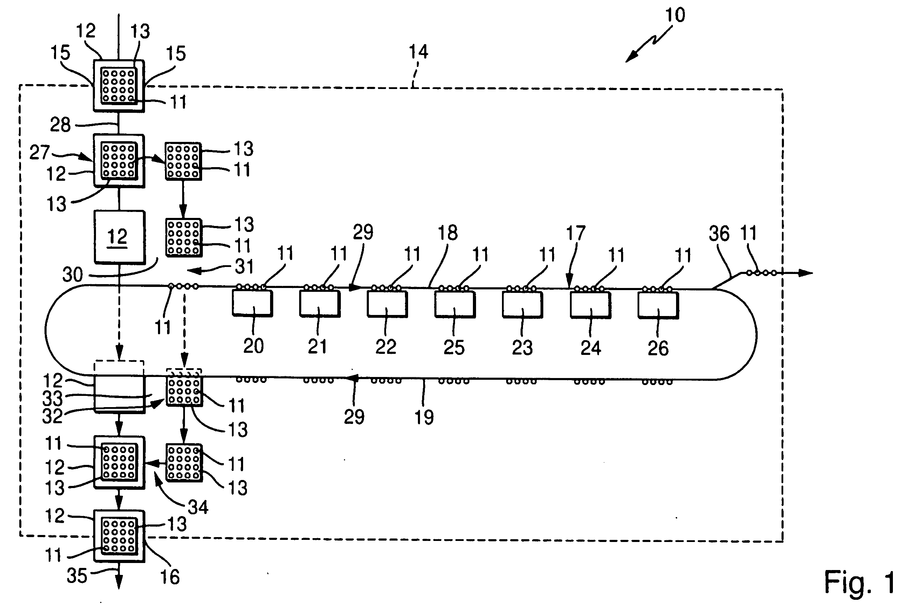

Die in Fig. 1 gezeigten Vorrichtung 10 ist für die Verarbeitung von vorsterilisierten Objekten 11 in aseptischer Weise ausgebildet. Sie ist zum Füllen und Verschließen solcher pharmazeutischer Objekte 11 bestimmt, die z. B. aus Spritzen, Vials, Zylinderampullen od. dgl. bestehen können.

Die Objekte 11 werden jeweils mittels etwa wannenförmiger Aufnahmebehälter 12, üblicherweise als "Tub" bezeichnet, zugefördert, wobei in den Tubs 12 mit Aufnahmen versehene, zumindest im Wesentlichen plattenförmige Aufnahmeelemente, üblicherweise als Nest 13 bezeichnet, eingesetzt sind, die aus den Tubs 12 entnehmbar sind bzw. in diese wiederum eingesetzt werden können. Die Objekte 11 sind in den Aufnahmen der Nester 13 in mehreren Reihen nebeneinander und hintereinander angeordnet, wobei die Reihen parallel zueinander verlaufen.

Die Vorrichtung 10 weist ein nur schematisch angedeutetes, z. B. etwa kastenartiges Gehäuse 14 auf, in dessen Innenraum ein Laminarflow erzeugt werden kann, der z. B. vom Deckenbereich des Gehäuses 14 in etwa vertikaler Richtung zum Boden gerichtet und mittels eines Gebläses erzeugt wird. Das Gehäuse 14 kann am Eingang bei 15 und am Ausgang bei 16 mit einer jeweiligen, nicht gezeigten Schleuse versehen sein. Im Inneren des Gehäuses 14 befindet sich eine mit Vorzug endlos arbeitende Fördereinrichtung 17, deren Transportstrecke einen dem Vorlauf dienenden geraden Abschnitt 18 und einen dem Rücklauf dienenden geraden Abschnitt 19 aufweist, der in Querabstand vom Abschnitt 18 verläuft, wobei beide Abschnitte 18, 19 am jeweiligen Ende durch Umlenkbögen verbunden sind. Entlang der Transportstrecke der Fördereinrichtung 17, insbesondere des Abschnitts 18 dieser, sind einzelne Bearbeitungsstationen für die Objekte 11 angeordnet, z. B. eine Wiegestation 20, eine Füllstation 21, eine zweite Wiegestation 22 zum Wiegen befüllter Objekte 11, eine Verschließstation 23 und eine Bördelstation 24. In der ersten Wiegestation 20 erfolgt das Wiegen der leeren, nicht befüllten Objekte 11. In der Füllstation 21 werden die Objekte 11 mittels Pharmazeutika gefüllt, die hinsichtlich Masse bzw. Volumen genau dosiert werden können. In der zweiten Wiegestation 22 werden die befüllten Objekte 11 gewogen. In der Verschließstation 23 werden die befüllten Objekte 11 verschlossen, wobei dies je nach Typ der Objekte 11 durch Einbringen von Verschlussstopfen erfolgen kann. In der Bördelstation 24 können aufgebrachte Bördelkappen 23 gebördelt werden. Gewünschtenfalls kann nach der Füllstation 11 und vor und/oder hinter der zweiten Wiegestation 22 noch eine Begasungsstation vorgesehen sein, z. B. eine angedeutete Begasungsstation 25 im Anschluss an die zweite Wiegestation 22. Im Anschluss an die Bördelstation 24 ist noch eine Schlechtausschubstation 26 angeordnet, bei der als schlecht erkannte Objekte 11 einzeln verworfen werden können. Nach Durchlaufen der einzelnen Bearbeitungsstationen im Bereich des Abschnitts 18, also des Vorlaufs, der Fördereinrichtung 17 werden die Objekte 11 über den Abschnitt 19, also Rücklauf, der Fördereinrichtung 17 wieder zurückgefördert. Die einzelnen Objekte 11 werden in Form einzelner Reihen in nicht weiter dargestellte Halter der Fördereinrichtung 17 gehängt und verbleiben während der Bearbeitung in den einzelnen erläuterten Stationen in dieser Position an der Fördereinrichtung 17. Der Abstand der Objekte 11 voneinander kann dabei gleich bleiben oder stattdessen auch verändert werden, z. B. gespreizt werden. In der jeweiligen Wiegestation 20 und 22 sind die Objekte 11 freigestellt, so dass diese nur Kontakt mit den Wiegetellern der Wiegestationen 20 bzw. 22 haben. Dies erlaubt beim Wiegen in der zweiten Wiegestation 22 eine exakte Kontrolle der Füllmengen. Beim Transport der Objekte 11 haben diese keinerlei Berührung miteinander. Beim Transport der Objekte 11 mittels der Fördereinrichtung 17 entlang den einzelnen Bearbeitungsstationen erfolgen praktisch keine Handhabungsbewegungen bei den Objekten 11, so dass etwaige sonst durch Handhabungsbewegungen und Angriff an den Objekten 11 bedingte Produktschädigungen nicht zu befürchten sind. Die in den Nestern 13 sortierten und orientiert vorliegenden Objekte 11 werden nach der Entnahme aus den Nestern 13 in den einzelnen Bearbeitungsstationen direkt bearbeitet. Dabei ergibt sich eine freie Sicht auf die Objekte 11, was optische Prüfverfahren, z. B. die Messung der Setztiefe eines in der Verschließstation 23 eingesetzten Stopfens, erlaubt.

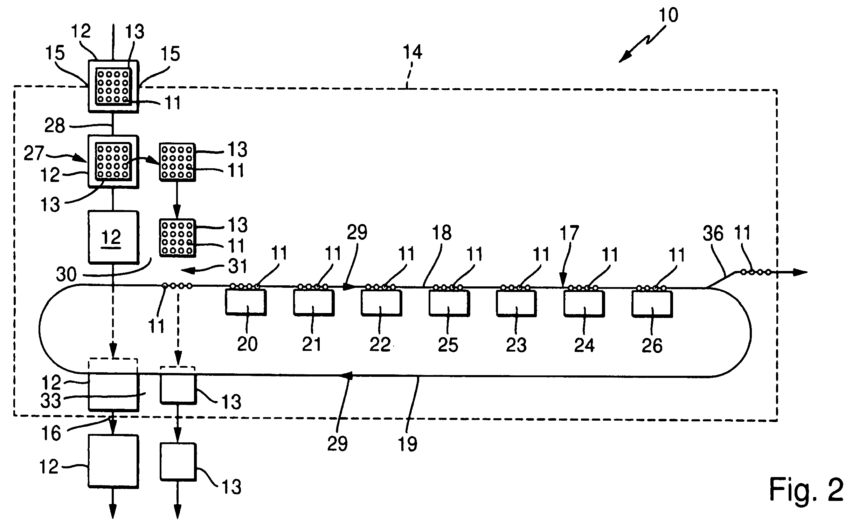

Man erkennt somit, dass die Vorrichtung 10 es gestattet, die Objekte 11 am Anfang der Fördereinrichtung 17 aus dem Nest 13 zu entnehmen, dann die Objekte 11 komplett mit allen Bearbeitungsschritten zu verarbeiten und am Ende entweder, wie in Fig. 1 gezeigt ist, wieder in das Nest 13 einzusetzen oder alternativ dazu entsprechend Fig. 2 am Ende vereinzelt aus der Fördereinrichtung 17 zu entfernen. Wenn die Objekte 11 gemäß Fig. 2, ohne die Umlenkung der Fördereinrichtung 17 zum rücklaufenden Abschnitt 19 zu durchlaufen und ohne Einsetzen ins Nest 13, direkt abgegeben werden, ergibt sich der Vorteil, dass für typischerweise nachfolgende Bearbeitungen, z. B. Etikettieren, die Objekte 11 nicht erneut aus dem Nest 13 gehoben werden müssen.

Die Vorrichtung 10 hat am Eingang 30 der Fördereinrichtung 17 eine schematisch angedeutete Aushebestation 27, in der aus über ein Transportband 28 z. B. quer, schräg oder längs zur Transportrichtung 29 der Fördereinrichtung 17 zugeführten, gefüllten Tubs 12 jeweils mit Objekten 11 besetzte Nester 13 entnommen werden, wobei die Nester 13 dem Eingang 30 der Fördereinrichtung 17 und einer dortigen Entnahmestation 31 zugeführt werden. In der Entnahmestation 31 werden die Objekte 11 in Reihen aus dem Nest 13 entnommen und an die Fördereinrichtung 17 übergeben und z. B. in dortige nicht gezeigte Halter gehängt. Die Fördereinrichtung 17 kann einen taktweisen oder kontinuierlichen Transport durchführen, wobei auch teilweise ein kontinuierlicher und teilweise ein taktweiser Transport in Betracht kommt, je nach Typ der jeweiligen Bearbeitungsstation. Die Objektreihen passieren zunächst die erste Wiegestation 20, in der die Objekte 11, zum Wiegen freigestellt, gewogen werden. Danach passieren die Objektreihen die Füllstation 21, in der das Füllen mit Pharmazeutika in dosierter Weise geschieht. Danach werden die Objektreihen zur zweiten Wiegestation 22 transportiert, in der die befüllten Objekte 11 erneut gewogen werden. Anschließend daran kann bedarfsweise in der Begasungsstation 25 ein Begasen der Objekte 11 erfolgen. In der anschließenden Verschließstation 23 werden z. B. bei Spritzenkörpem als Objekte 11 oder auch bei Vials als solche Verschlussstopfen zum Verschließen eingebracht. Bei Vials als Objekte 11 oder auch bei Zylinderampullen werden anschließend in der Bördelstation 24 Bördelkappen aufgebracht und gebördelt.

Die Vorrichtung 10 kann an verschiedenen Stellen Prüfstationen, z. B. Kameras, aufweisen, mittels denen eine jeweilige Prüfung der Objekte 11 im jeweiligen Bearbeitungsstadium möglich ist. Ergibt eine Prüfung Mängel, so können die Objekte 11 bei der Schlechtausschubstation 26 von der Fördereinrichtung 17 z. B. einzeln und stellenbezogen entfernt und ausgeschoben werden. Für gut befundene Objekte 11 gelangen vor oder nach Umlenkung, z. B. mittels des Abschnitts 19 des Rücklaufs der Fördereinrichtung 17, zu einer dortigen Einsetzstation 32, in der die befüllten Objekte 11 wieder in die Nester 13 eingesetzt werden. Die am Eingang 30 bei der Entnahmestation 31 entleerten Nester 13 werden gleich nach Ausheben der Objekte 11 z.B. unterhalb der Fördereinrichtung 17, quer zur Transportrichtung gemäß Pfeil 29, zum Ausgang 33 oder zu einer anderen Ausgangsseite gefördert werden. Die Nester 13 können dann entweder gleich in die Tubs 12 eingesetzt werden, so dass die bearbeiteten Objekte 11 in die bereits in den Tubs 12 enthaltenen Nester 13 eingebracht werden können, oder die Nester 13 werden zunächst zur Einsetzstation 32 transportiert, wo dann die gefüllten und gewogenen Objekte 11 in die Nester 13 eingebracht werden. Die Tubs 12 können nach Entnahme der Nester 13 im Bereich der Aushebestation 27, z. B. unterhalb der Fördereinrichtung 17, z. B. quer oder schräg oder längs zur Transportrichtung gemäß Pfeil 29, zur Ausgangsseite hin und dort zu einer Einsetzstation 34 transportiert werden, in der die leeren oder mit befüllten Objekten 11 versehenen Nester 13 in die Tubs 12 eingesetzt werden. Diese verlassen dann den Ausgang 16 in Richtung quer oder schräg oder längs zur Transporteinrichtung gemäß Pfeil 35.

Man erkennt, dass die Objekte 11 nach Entnahme aus dem Nest 13 in der Entnahmestation 31 in den einzelnen Bearbeitungsstationen komplett mit allen Bearbeitungsschritten verarbeitet werden und erst am Ende wieder in die Nester 13 zurückgegeben werden und diese schon gleich nach Entleeren oder nach Befüllung mit den Objekten 11 in die Tubs 12 eingesetzt werden und letztere die Vorrichtung 10 verlassen. Je mehr Bearbeitungsstationen bei der Vorrichtung 10 vorhanden sind, umso vorteilhafter ist diese Verfahrensweise, bei der die Objekte 11 einmal am Anfang aus dem Nest 13 entnommen werden und erst nach Durchlauf aller Bearbeitungsschritte wieder in die Nester 13 zurückgesetzt werden. Eine vielfache Berührung der Objekte 11 wird vermieden. Da im Wesentlichen keine Handhabungsbewegungen durch Angreifen an den Objekten 11 erfolgen, ergeben sich weniger Produktschädigungen.

Bei dem in Fig. 2 gezeigten zweiten Ausführungsbeispiel sind die Teile, die dem ersten Ausführungsbeispiel in Fig. 1 entsprechen, gleiche Bezugszeichen verwendet.

Das zweite Ausführungsbeispiel gemäß Fig. 2 unterscheidet sich von demjenigen in Fig. 1 dadurch, dass die befüllten Objekte 11 nach Passieren der letzten Bearbeitungsstation, z. B. der Schlechtausschubstation 26, vereinzelt von der Fördereinrichtung 17 längs einer Bahn 36 abgegeben werden, die längs, schräg oder quer zur Transportrichtung gemäß Pfeil 29 verläuft. Die Bahn 36 kann auch beim Ausführungsbeispiel gemäß Fig. 1 für den Abtransport von als mangelhaft erkannten Objekten in der Schlechtausschubstation 26 vorgesehen sein. Die Tubs 12, die nach der Aushebestation 27 leer sind, werden quer zur Fördereinrichtung 17 z. B. unterhalb dieser abtransportiert und gestapelt oder verworfen. In gleicher Weise werden auch die Nester 13, die nach Entnahme der Objekte 11 im Bereich der Entnahmestation 31 leer geworden sind, quer zur Fördereinrichtung 17, z. B. unterhalb dieser, abtransportiert und gestapelt oder verworfen. Die über die Bahn 36 abtransportierten Objekte 11 können vereinzelt an nachfolgende Anlagen, z. B. zur Inspektion, übergeben werden, wobei der Auslauf z. B. vor oder hinter der Umlenkung und dabei quer oder schräg oder längs zur Transportrichtung gemäß Pfeil 29 erfolgen kann.

## Patentansprüche

1. Verfahren zum Füllen und Verschließen von pharmazeutischen Objekten (11), z. B. Spritzen, Vials, Zylinderampullen od. dgl., die in parallelen Reihen in Nestern (13) angeordnet sind, mit folgenden Verfahrensschritten:
a) die Objekte (11) werden an einem Eingang (30) einer Fördereinrichtung (17) in einer Entnahmestation (31) den Nestern (13) entnommen und in die Fördereinrichtung (17) eingebracht,
b) mittels der Fördereinrichtung (17) werden die Objekte (11) entlang eines geraden Abschnitts (18) der Transportstrecke nacheinander einer ersten Wiegestation (20), in der die Objekte (11) gewogen werden, einer Füllstation (21), in der die Objekte (11) befüllt werden, und einer zweiten Wiegestation (22) zugeführt, in der die befüllten Objekte (11) wiederum gewogen werden,
c) danach werden die befüllten Objekte (11) einer Verschließstation und/oder Bördelstation (24) zugeführt, in der die befüllten Objekte (11) verschlossen werden,
d) die befüllten Objekte (11) werden aus der Transportstrecke abgegeben, wobei die Bearbeitung der vorsterilisierten Objekte (11) vorzugsweise aseptisch erfolgt,
wobei der gerade Abschnitt (18) der Transportstrecke zu einem geraden Rücklaufabschnitt (19) umgelenkt wird und die befüllten Objekte (11) zu einem Ausgang (33) zurückgeführt werden, der vor oder nach der Umlenkung vorgesehen ist, und wobei die Objekte (11) am Eingang (30) der Fördereinrichtung (17) aus den Nestern (13) entnommen werden, wobei die Nester (13) am Eingang (30) der Fördereinrichtung (17) von der Entnahmestation (31) quer zu dem geraden Abschnitt (18) der Transportstrecke abtransportiert werden, wobei dann die Objekte (11) komplett mit allen Bearbeitungsschritten in dem geraden Abschnitt (18) verarbeitet werden, und wobei die Objekte (11) an dem Ausgang (33) wieder in die Nester (13) eingesetzt oder vereinzelt, ohne die Umlenkung der Fördereinrichtung (17) zu dem rücklaufenden Abschnitt (19) zu durchlaufen aus der Fördereinrichtung (17) entfernt werden.

2. Verfahren nach Anspruch 1,
**dadurch gekennzeichnet, dass**
die befüllten Objekte (11) zu einer Einsetzstation (32) zurückgeführt werden, in der die Objekte (11) in die Nester (13) eingesetzt werden.

3. Verfahren nach Anspruch 2,
**dadurch gekennzeichnet, dass**
die Nester (13) mit enthaltenen befüllten Objekten (11) in Bezug auf die Fördereinrichtung (17) abgeführt werden.

4. Verfahren nach einem der Ansprüche 1 bis 3,
**dadurch gekennzeichnet, dass**
in Tubs (12) enthaltene Nester (13) in einer Aushebestation (27) am Eingang (30) der Fördereinrichtung (17) aus den Tubs (12) entnommen werden und beim Rücklauf in einer Einsetzstation (34) wieder in die Tubs (12) eingesetzt werden oder vor dem Rücklauf oder bei diesem abtransportiert werden.

5. Verfahren nach einem der Ansprüche 1 bis 4,
**dadurch gekennzeichnet, dass**
die nicht befüllten Objekte (11) in Reihen den Nestern (13) entnommen und im befüllten Zustand entweder in Nester (13), die dann abgegeben werden, eingesetzt werden oder vor oder nach der Umlenkung aus der Transportstrecke abgegeben werden.

6. Verfahren nach Anspruch 4,
**dadurch gekennzeichnet, dass**
die Tubs (12) und/oder Nester (13) am Eingang (30) der Fördereinrichtung (17) von der Aushebestation (27) bzw. von der Entnahmestation (31), z. B. quer zur Transportstrecke, zum dortigen Bereich (19) des Rücklaufabschnitts der Transportstrecke transportiert werden und zu einer dortigen Einsetzstation (34) für die Nester (13), in der die mit befüllten Objekten (11) versehenen Nester (13) in die Tubs (12) eingesetzt werden, bzw. zu einer dortigen Einsetzstation (32) für die befüllten Objekte (11) zurückgeführt werden, in der die befüllten Objekte (11) in Nester (13) eingesetzt werden.

7. Verfahren nach einem der Ansprüche 1 bis 6,
**dadurch gekennzeichnet, dass**
die befüllten Objekte (11) entlang der Transportstrecke nach der Füllstation (21) und vor und/oder hinter der zweiten Wiegestation (22) einer Begasungsstation (25) zugeführt werden.

8. Verfahren nach einem der Ansprüche 1 bis 7,
**dadurch gekennzeichnet, dass**
die befüllten Objekte (11) in der Verschließstation (23) mit einem Verschlussstopfen versehen und/oder bedarfsweise in der Bördelstation (24) gebördelt werden.

9. Verfahren nach einem der Ansprüche 1 bis 8,
**dadurch gekennzeichnet, dass**
die als schlecht erkannten Objekte (11) an einer Schlechtausschubstation (26) einzeln und stellenbezogen verworfen werden.

10. Vorrichtung zum Füllen und Verschließen von pharmazeutischen Objekten (11), z. B. Spritzen, Vials, Zylinderampullen od. dgl., wobei die Objekte (11) in parallelen Reihen in Nestern (13) angeordnet sind, die in einen etwa wannenförmigen Tub (12), daraus entnehmbar, eingesetzt sind, mit einer Fördereinrichtung (17), entlang deren Transportstrecke einzelne Bearbeitungsstationen für die Objekte (11) angeordnet sind, insbesondere zum Wiegen, Befüllen und Verschließen der Objekte (11), wobei am Eingang (30) der Fördereinrichtung (17) eine Aushebestation (27) vorgesehen ist zum Entnehmen des Nestes (13) aus dem Tub (12), auf die eine Entnahmestation (31) zum Entnehmen einer Objektreihe aus dem Nest (13) und Übergeben dieser an die Fördereinrichtung (17) folgt,
**dadurch gekennzeichnet, dass**
die Fördereinrichtung (17) einen geraden Abschnitt (18) aufweist, der zu einem geraden Rücklaufabschnitt (19) umgelenkt wird, wobei die Nester (13) am Eingang (30) der Fördereinrichtung (17) von der Entnahmestation (31) quer zu dem geraden Abschnitt (18) der Transportstrecke abtransportiert werden, und
dass die aus dem Nest (13) entnommenen Objekte (11) mittels der Fördereinrichtung (17) den einzelnen Bearbeitungsstationen (20, 21, 22, 23, 24, 26) in dem geraden Abschnitt (18) zugeführt und dort mit allen Bearbeitungsschritten bearbeitet werden und am Ende entweder in einer Einsetzstation (32) in das Nest (13) gesetzt werden, welches in einer Einsetzstation (34) entweder vorher bereits in das Tub (12) eingesetzt worden ist oder anschließend in das Tub (12) eingesetzt wird, oder ohne die Umlenkung der Fördereinrichtung (17) zu dem rücklaufenden Abschnitt (19) zu durchlaufen vereinzelt von der Fördereinrichtung (17) abgegeben werden.

## Claims

1. A method for filling and closing pharmaceutical objects (11), e.g. syringes, vials, cylindrical ampules or else, disposed in parallel rows in nests (13), comprising steps of:
a) in a removal station (31), at an inlet (30) of a conveying device (17), removing the objects (11) from the nests (13) and placing the objects (11) in the conveying device (17);
b) by the conveying device (17), feeding the objects (11) in succession along a straight section (18) of the conveying way to a first weighing station (20) wherein the objects (11) are weighed, a filling station (21) wherein the objects (11) are filled, and a second weighing station (22) wherein the filled objects (11) are again weighed;
c) afterwards, feeding the objects to a closing station (23) and/or a crimping station (24) in which the filled objects (11) are closed;
d) removing the filled objects (11) from the conveying section, wherein processing of pre-sterilized objects (11) is preferably implemented aseptically,
wherein the straight section (18) of the conveying way is redirected to a straight return section (19) and the filled objects (11) are fed back to an outlet (33) which is provided upstream or downstream of the redirection and wherein the objects (11) are lifted out of the nests (13) at the inlet (30) of the conveying device (17), wherein the nests (13) at the inlet (30) of the conveying device (17) are conveyed away from the removal station (31) transversely to the conveying way, wherein the objects (11) are then processed completely with all processing steps in the straight section (18) of the conveying way, and wherein the objects (11) are either returned to the nests (13) at the outlet (33) or removed individually from the conveying device (17) without running through the redirection of the conveying device (17) to the returning section (19).

2. The method according to claim 1,
**characterized in that**
the filled objects (11) are fed back to an insertion station (32), in which the filled objects (11) are placed in the nests (13).

3. The method according to claim 2,
**characterized in that**
the nests (13) are lead away relative to the conveying device (17) containing the filled objects (11).

4. The method according to any of claims 1 to 3,
**characterized in that**
nests (13) contained in tubs (12) are removed from the tubs (12) in a lifting station (27) at the inlet (30) of the conveying device (17) and, in an insertion stations (34), the nests (13) are returned to the tubs (12) during return or the nests (13) are away upstream of or at the return.

5. The method according to any of claims 1 to 4,
**characterized in that**
the non-filled objects (11) are removed from the nests (13) in rows and, in the filled state, are either placed in nests (13), which are then dispensed, or are removed from the conveying device (17) upstream or downstream of the redirection.

6. The method according to claim 4,
**characterized in that**
the tubs (12) and/or nests (13) at the inlet (30) of the conveying device (17) are conveyed from the lifting station (27) or from the removal station (31), e.g. transversely to the conveying way, to a region of the return section (19) of the conveying way and are conveyed to the insertion station (34) there for the nests (13), in which the nests (13) provided with filled objects (11) are placed into the tube (12) or are returned to an insertion station (32) there for the filled objects (11), in which the filled objects (11) are placed in nests (13).

7. The method according to any of claims 1 to 6,
**characterized in that**
the filled objects (11) are fed to a gas-treatment station (25) along the conveying way downstream of the filling station (21) and upstream and/or downstream of the second weighing station (22).

8. The method according to any of claims 1 to 7,
**characterized in that**
the filled objects (11) in the closing station (23) are provided with a stopper and/or are crimped in the crimping station (24), if necessary.

9. The method according to any of claims 1 to 8,
**characterized in that**
the filled objects (11) that are identified as rejects are discarded at a reject-discarding station (26) individually and in a position-specific manner.

10. A device for filling and closing pharmaceutical objects (11), e.g. syringes, vials, cylindrical ampules or else, wherein the objects (11) are disposed in parallel rows in nests (13), which nests (13) are removably inserted into a substantially tub-shaped tub (12), the device comprising a conveying device (17), wherein individual processing stations (20, 21, 22, 23, 24, 26) for the objects (11) are disposed along a conveying way of the conveying device (17), in particular wherein the individual processing stations (20, 21, 22, 23, 24, 26) are for weighing, filling and closing the objects (11), wherein a lifting station (27) is provided at an inlet (30) of the conveying device (17), the lifting station (27) for removing the nests (13) from the tub (12), a removal station (31) for removing an object row from the nest (13) and transferring the object row to the conveying device (17) following the lifting station (27),
**characterized in that**
the conveying device (17) has a straight section (18) which is redirected to a straight return section (19), wherein the nests (13) at the inlet (30) of the conveying device (17) are conveyed away from the removal station (31) transversely to the conveying way, and
**in that** the objects (11) removed from the nest (13) are fed by the conveying device (17) to the individual processing stations (20, 21, 22, 23, 24, 28) in the straight section (18) and are there processed with all processing steps and are either placed in an insertion station (32) into the nest (13) that has either already been inserted into the tub (12) in an insertion station (34) or is subsequently inserted into the tub (12), or are removed individually from the conveying device (17) without running through the redirection of the conveying device (17) to the returning section (19).

## Revendications

1. Procédé pour remplir et fermer des objets pharmaceutiques (11), par exemple des seringues, des fioles, des ampoules cylindriques ou similaires, qui sont disposés en rangées parallèles dans des alvéoles (13), comprenant les étapes suivantes :
a) les objets (11) sont prélevés des alvéoles (13) à une entrée (30) d'un dispositif de convoyage (17) dans une station de prélèvement (31) et introduits dans le dispositif de convoyage (17),
b) les objets (11) sont acheminés au moyen du dispositif de convoyage (17), le long d'une portion droite (18) du trajet de transport, l'un derrière l'autre à une première station de pesage (20) dans laquelle les objets (11) sont pesés, à une station de remplissage (21) dans laquelle les objets (11) sont remplis et à une deuxième station de pesage (22) dans laquelle les objets (11) remplis sont une nouvelle fois pesés,
c) les objets (11) remplis sont ensuite acheminés à une station de fermeture et/ou une station de bordage (24) dans laquelle les objets (11) remplis sont fermés,
d) les objets (11) remplis sont déchargés du trajet de transport, le traitement des objets (11) pré-stérilisés étant de préférence réalisé de manière aseptisée, la portion droite (18) du trajet de transport étant renvoyée vers une portion de retour droite (19) et les objets (11) remplis étant retournés vers une sortie (33) qui est prévue avant ou après le renvoi, et les objets (11) étant prélevés hors des alvéoles (13) à l'entrée (30) du dispositif de convoyage (17), les alvéoles (13) à l'entrée (30) du dispositif de convoyage (17) de la station de prélèvement (31) étant emportés transversalement à la portion droite (18) du trajet de transport, les objets (11) étant ensuite entièrement traités dans la portion droite (18) avec toutes les étapes de traitement, et les objets (11) au niveau de la sortie (33) étant de nouveau introduits dans les alvéoles (13) ou retirés individuellement hors du dispositif de convoyage (17) sans passer à travers le renvoi du dispositif de convoyage (17) vers la portion de retour (19).

2. Procédé selon la revendication 1, **caractérisé en ce que** les objets (11) remplis sont retournés vers une station d'introduction (32) dans laquelle les objets (11) sont introduits dans les alvéoles (13).

3. Procédé selon la revendication 2, **caractérisé en ce que** les alvéoles (13) qui contiennent les objets (11) remplis sont évacuées par rapport au dispositif de convoyage (17).

4. Procédé selon l'une des revendications 1 à 3, **caractérisé en ce que** les alvéoles (13) contenues dans des tubes (12) sont prélevées des tubes (12) dans une station de levage (27) à l'entrée (30) du dispositif de convoyage (17) et, lors du retour, sont de nouveau introduits dans les tubes (12) dans une station d'introduction (34) ou évacués avant le retour ou au niveau de celui-ci.

5. Procédé selon l'une des revendications 1 à 4, **caractérisé en ce que** les objets (11) non remplis sont prélevés dans les rangées des alvéoles (13) et, à l'état rempli, sont soit de nouveau introduits dans les alvéoles (13), lesquelles sont ensuite déposées, soit déposés du trajet de transport avant ou après le renvoi.

6. Procédé selon la revendication 4, caractérisé en ce les tubes (12) et/ou les alvéoles (13) à l'entrée (30) du dispositif de convoyage (17) sont transportés de la station de levage (27) ou de la station de prélèvement (31), par exemple transversalement par rapport au trajet de transport, vers la zone (19) qui s'y trouve de la portion de retour du trajet de transport, puis sont retournés vers une station d'introduction (34) pour les alvéoles (13) qui s'y trouve, dans laquelle les alvéoles (13) munies des objets (11) remplis sont introduites dans les tubes (12), ou vers une station d'introduction (32) pour les objets (11) remplis qui s'y trouve, dans laquelle les objets (11) remplis sont introduits dans les alvéoles (13).

7. Procédé selon l'une des revendications 1 à 6, **caractérisé en ce que** les objets (11) remplis sont acheminés à une station de fumigation (25) le long du trajet de transport après la station de remplissage (21) et devant et/ou derrière la deuxième station de pesage (22).

8. Procédé selon l'une des revendications 1 à 7, **caractérisé en ce que** les objets (11) remplis sont munis d'un bouchon de fermeture dans la station de fermeture (23) et/ou, au besoin, sont bordés dans la station de bordage (24).

9. Procédé selon l'une des revendications 1 à 8, **caractérisé en ce que** les objets (11) identifiés comme non conformes sont éjectés individuellement et en référence à leur position au niveau d'une station d'éjection des non conformes (26).

10. Dispositif pour remplir et fermer des objets pharmaceutiques (11), par exemple des seringues, des fioles, des ampoules cylindriques ou similaire, les objets (11) étant disposés en rangées parallèles dans des alvéoles (13) qui sont introduites dans un tube (12) approximativement en forme de cuve duquel ils peuvent être prélevés, comprenant un dispositif de convoyage (17) le long du trajet de transport duquel sont disposées des stations de traitement individuelles pour les objets (11), notamment pour le pesage, le remplissage et la fermeture des objets (11), une station de levage (27) étant prévue à l'entrée (30) du dispositif de convoyage (17) pour prélever l'alvéole (13) hors du tube (12), laquelle est suivie par une station de prélèvement (31) destinés à prélever une rangée d'objets hors de l'alvéole (13) et à transférer celle-ci au dispositif de convoyage (17),
**caractérisé en ce que**
le dispositif de convoyage (17) possède une portion droite (18) qui est renvoyées vers une portion de retour (19) droite, les alvéoles (13) à l'entrée (30) du dispositif de convoyage (17) étant emportés de la station de prélèvement (31) transversalement à la portion (18) droite du trajet de transport, et
**en ce que** les objets (11) prélevés des alvéoles (13) sont acheminés au moyen du dispositif de convoyage (17) aux stations de traitement (20, 21, 22, 23, 24, 26) individuelles dans la portion (18) droite et y sont traités avec toutes les étapes de traitement puis, à la fin, sont soit de nouveau introduits dans l'alvéole (13) dans une station d'introduction (32), alvéole (13) qui a soit déjà été préalablement introduite dans le tube (12) dans une station d'introduction (34), soit qui est introduite ensuite dans le tube (12), soit sont retirés individuellement hors du dispositif de convoyage (17) sans passer à travers le renvoi du dispositif de convoyage (17) vers la portion de retour (19).
